# EUROPEAN PATENT SPECIFICATION

(11) **EP 2 327 684 B1**
(45) Date of publication and mention of the grant of the patent: **07.10.2015**
(21) Application number: 10191442.2
(22) Date of filing: 16.11.2010
(51) Int. Cl.: C07C 45/29, C07C 209/28, C07C 209/84

(54) **A process for the preparation of cinacalcet and intermediates thereof**
Verfahren zur Herstellung von Cinacalcet und Zwischenprodukten davon
Procédé pour la préparation de cinacalcet et intermédiaires de cette préparation

(30) Priority: 26.11.2009 IT MI20092079
(43) Date of publication of application: 01.06.2011
(73) Proprietor: Dipharma Francis S.r.l., 20021 Baranzate (MI) (IT)
(72) Inventor: Allegrini, Pietro, 20097 S. Donato Milanese (MI) (IT); Attolino, Emanuele, 74019 Palagiano (TA) (IT); Rossi, Davide, 21023 Malgesso (VA) (IT)
(74) Representative: Minoja, Fabrizio

(56) References cited:
- WO-A2-2008/058235
- US-B1- 6 211 244
- WANG X ET AL: "Synthesis of Cinacalcet congeners", TETRAHEDRON LETTERS, ELSEVIER, AMSTERDAM, NL LNKD- DOI:10.1016/J.TETLET.2004.09.063, vol. 45, no. 45, 1 November 2004 (2004-11-01), pages 8355-8358, XP004593872, ISSN: 0040-4039
- SORBERA L A ET AL: "Cinacalcet hydrochloride", DRUGS OF THE FUTURE, PROUS SCIENCE, ES, vol. 27, no. 9, 1 January 2002 (2002-01-01), pages 831-836, XP002403819, ISSN: 0377-8282, DOI: DOI:10.1358/DOF.2002.027.09.695016
- NAN JIANG ABD ARTHUR J. RAGAUSKAS: "Cu(II)-Catalyzed Selective Aerobic Oxidation of Alcohols under Mild Conditions", JOURNAL OF ORGANIC CHEMISTRY, vol. 71, no. 18, 2006, pages 7087-7090, XP002621654, DOI: 10.1021/jo060837y
- BLEICHER K H ET AL: "Parallel synthesis of substituted imidazoles from 1,2-aminoalcohols", TETRAHEDRON LETTERS, ELSEVIER, AMSTERDAM, NL, vol. 43, no. 43, 21 October 2002 (2002-10-21), pages 7687-7690, XP004385652, ISSN: 0040-4039, DOI: DOI:10.1016/S0040-4039(02)01839-7
- CLAYDEN J ET AL: "(S)-2-(Dibenzylamino)-3-phenylpropanal as a chiral auxiliary: a new strategy for the asymmetric synthesis of 2-substituted alcohols", TETRAHEDRON ASYMMETRY, PERGAMON PRESS LTD, OXFORD, GB, vol. 9, no. 8, 24 April 1998 (1998-04-24), pages 1427-1440, XP004131400, ISSN: 0957-4166, DOI: DOI:10.1016/S0957-4166(98)00120-7
- BUSSEY C ET AL: "Double-headed molecules based on linear or angular psoralen and alpha-methylene-psi-butyrolactone: a search for non-phototoxic potential antiproliferative compounds", BIOORGANIC & MEDICINAL CHEMISTRY LETTERS, PERGAMON, ELSEVIER SCIENCE, GB, vol. 3, no. 6, 1 June 1993 (1993-06-01), pages 1283-1286, XP026679597, ISSN: 0960-894X, DOI: 10.1016/S0960-894X(00)80332-7 [retrieved on 1993-06-01]
- DOUGLAS F. TABER ET AL: "P2O5/DMSO/Triethylamine (PDT): A convenient Procedure for Oxidation of Alcohols to Ketones and Aldehydes", JOURNAL OF ORGANIC CHEMISTRY, vol. 52, 1987, pages 5621-5622,
- KONOSHIN ONODERA ET AL: "Oxidation of Carbohydrates with Dimethyl Sulfoxide Containing Phosphorus Pentoxide", JOURNAL OF THE AMERICAN CHEMICAL SOCIETY, vol. 87, 1965, pages 4651-4652,
- Section 2.4.: "Albright-Onodera Oxidation (Phosphorous Pentoxide-Mediated Moffatt Oxidation)" In: Tojo, G.; Fernandez, M.I.: "Oxidation of Alcohols to Aldehydes and Ketones", 2006, Springer ISBN: 978-0-387-23607-0 pages 118-120, * the whole document *

## Description

### FIELD OF THE INVENTION

The present invention relates to a process for the preparation of (*R*)-(1-Naphthalen-1-yl-ethyl)-[3-(3-trifluoromethyl-phenyl)-propyl]-amine or a salt thereof, in particular the hydrochloride, and intermediates useful in its synthesis.

### TECHNOLOGICAL BACKGROUND

Cinacalcet, namely (*R*)-(1-Naphthalen-1-yl-ethyl)-[3-(3-trifluoromethyl-phenyl)-propyl]-amine of formula **(I),** is a known compound with anti-hyperparathyroid action, marketed as the hydrochloride.

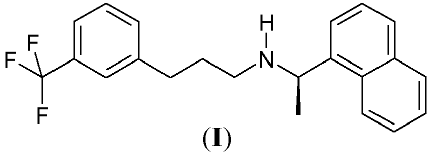

US 6,211,244 discloses its synthesis by condensation of 1-acetyl naphthalene and 3-[3-(trifluoromethyl)phenyl]propylamine in the presence of titanium isopropoxide and subsequent reduction of the resulting imine with sodium cyanoborohydride. The obtained compound (racemic Cinacalcet base) is then resolved by separation of the enantiomers with a chiral chromatographic column.

Drugs of the Future 2002, 27(9), 832 reports a similar process for the preparation of Cinacalcet starting from 3-[3-(trifluoromethyl)phenyl]-propionaldehyde of formula (**II**) 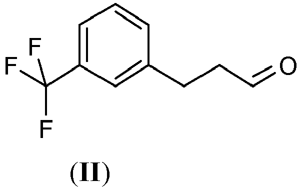 and (*R*)-1-(1-naphthyl)ethylamine of formula (**III**) 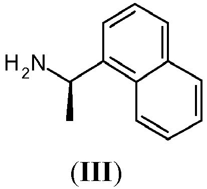 by formation of an imine intermediate and subsequent reduction with sodium cyanoborohydride.

It can be noticed that said processes either make use of toxic reagents, such as sodium cyanoborohydride, or involve the resolution of the racemate, which remarkable affects costs.

On the other hand, WO 06/125026 suggests the synthesis of Cinacalcet of formula (**I**) by reaction between an alkylating agent of formula (**IV**) derived from 3-[3-(trifluoromethyl)phenyl]propanol 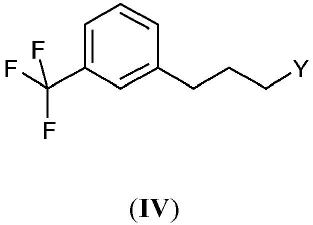 wherein Y is a leaving group, and (*R*)-1-(1-naphthyl)ethylamine of formula (**III**).

Said process involves the use of a large excess of alkylating agent (**IV**) to obtain Cinacalcet free from (*R*)-1-(1-naphthyl)ethylamine (**III**), which negatively affects both costs and production times, and is therefore poorly suited to the industrial application.

Furthermore, the preparation of a compound of formula (**II**) from the compound of formula (**V**) 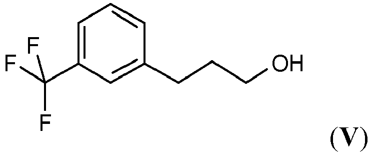 often results in low or not reproducible yields.

In this regard, the process disclosed in Tetrahedron Letters 2004, 45(45), pages 8355-8358 to prepare a congener of a compound of formula (**II**) suffers from the above mentioned drawbacks. Moreover, oxidation processes on a variety of alcoholic substrates disclosed in "Oxidation of alcohols to aldehydes and ketones" section 2.4: "Albright-Onodera Oxidation (Phosphorous Pentoxide-Mediated Moffatt Oxidation)", Springer, 2006, pages 118-120 or in Journal of American Chemical Society 1965, 87, pages 4651-4652 or in Journal of Organic Chemistry 1987, 52, pages 5621-5622 or in Bioorganic & Medicinal Chemistry Letters 1993, vol. 3, no. 6, pages 1283-1286 also suffer from the above mentioned drawbacks.

There is therefore the need for an alternative synthetic route which provides Cinacalcet or a salt thereof in high purity, from low cost starting materials.

### SUMMARY OF THE INVENTION

An alternative process has been found which allows to obtain Cinacalcet or a salt thereof starting from non toxic, inexpensive starting materials and with few synthetic steps. This makes the process of the invention more advantageous than the known processes.

### DETAILED DISCLOSURE OF THE INVENTION

Object of the invention is a process for preparing a compound of formula (**I**) or a salt thereof 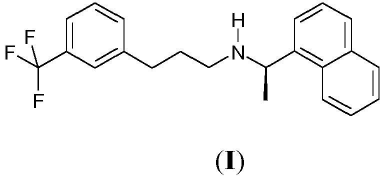 comprising the reductive amination of a compound of formula (**II**) 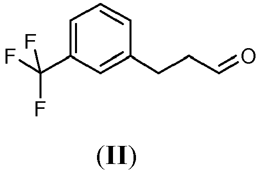 by reaction with (*R*)-1-(1-naphthyl)ethylamine of formula (**III**) or a salt thereof 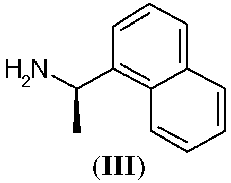 in the presence of a selective reducing agent of the imines.

A selective reducing agent of the imines is for example selected from the group comprising sodium triacetoxyborohydride; the InCl₃/triethylsilane system; the Ti(iPrO)₄/polymethylhydrosiloxane system; Bu₂SnC1H; Bu₂SnIH; the PhMe₂SiH/B(C₆F₅)₃ system; Zn(BH₄)₂/silica gel; NiCl₂/NaBH₄ and hydrogen in catalytic hydrogenation conditions; preferably sodium triacetoxyborohydride.

A salt of a compound of formula (**I**) or (**III**) is preferably a pharmaceutically acceptable salt, for example the hydrochloride, maleate, sulfate, phosphate, sulfamate, acetate, citrate and tartrate, preferably the hydrochloride salt.

The InCl₃/triethylsilane system can be in the presence of a Fe(**II**) or Zn(**II**) salt, for example FeSO₄.

The selective reducing agents of the imines reported above are commercially available and/or known. For example, sodium triacetoxyborohydride is commercially available or can be prepared *in situ* starting from acetic acid and sodium borohydride.

The catalytic hydrogenation can be carried out with hydrogen and a homogeneous or heterogeneous metal catalyst, based on palladium, platinum, nickel, rhodium or ruthenium, using hydrogen under a pressure which can be comprised between about 1 atm and 20 atm.

When the metal catalyst is heterogeneous, it is preferably deposited on an inert carrier, such as charcoal, barium hydroxide, alumina, calcium carbonate, preferably charcoal. The concentration of the metal on the carrier can be comprised between about 1% and 30%, preferably between 5% and 10%.

The reductive amination is preferably carried out in presence of a solvent selected from a dipolar aprotic solvent, typically dimethylformamide, dimethylacetamide, acetonitrile and dimethylsulfoxide; an ether, typically tetrahydrofuran or dioxane; a straight or branched C₁-C₆ alkanol, preferably a straight or branched C₁-C₄ alkanol such as methanol, ethanol or isopropanol; a C₁-C₆ carboxylic acid such as acetic acid or propionic acid; or a mixture of two or more, preferably two or three, of said solvents; or in water or in an aqueous solution of a protic organic or mineral acid such as trifluoroacetic acid, methanesulfonic and hydrochloric acid, or a mixture thereof with one or more, preferably two or three, of said solvents.

After completion of the reaction, the compound of formula (**I**) can be recovered as the free base, by means of known methods.

A pharmaceutically acceptable salt of a compound of formula (**I**) can be obtained by known methods. For example, a salt thereof can be obtained dissolving the free base in ethyl acetate and then reacting it with a protic organic acid, for example acetic, citric, tartaric, fumaric or succinic acid, or with a protic mineral acid, for example hydrochloric, hydrobromic or sulphuric acid, preferably aqueous or gaseous hydrochloric acid.

The recovery of a salt of a compound of formula (**I**), for example the hydrochloride, can be carried out according to known methods, for example by filtration.

According to a preferred embodiment of the invention, a salt of a compound of formula (**I**) can be obtained by recrystallization from a solvent in which said salt is poorly soluble at a temperature comprised between about - 10°C and 20°C, whereas the same salt is soluble at a temperature comprised between about 20°C and the reflux temperature of the crystallization solvent. Typically, a crystallization solvent can be selected from isopropanol, ethyl acetate or an acetonitrile/water mixture.

Cinacalcet hydrochloride, when obtained according to these crystallization conditions, has a XRPD wherein the more intense peaks correspond to those reported in figure 1 of WO 06/127933, which is the crystalline form I.

The size of the crystals of a compound of formula (**I**) or of a salt thereof, obtained according to the invention, is characterized by a D₅₀ value comprised between about 25 and 250 µm. If desired, said value can be reduced by micronisation or fine grinding, according to known methods.

The resulting Cinacalcet hydrochloride has purity equal to or higher than 99.5%, typically equal to or higher than 99.9%.

A compound of formula (**III**) or a salt thereof is commercially available. An acid addition salt of a compound (**III**) can be prepared from the corresponding free base by treatment with a protic organic or mineral acid, as defined above, according to known procedures.

Known methods for the preparation of an aldehyde of formula (**II**) 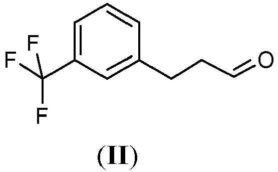 comprise the selective Swern oxidation of the corresponding alcohol (V) 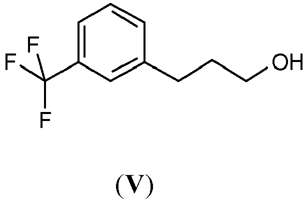 with oxalyl chloride (COCl)₂ and dimethylsulfoxide (DMSO) as reported in Tetrahedron Letters 2004, 45(45), 8355-8358. Said Swern oxidation is a remarkable problem from the industrial point of view, mainly in that it makes use of oxalyl chloride which is unstable and reaction conditions, such as low reaction temperature (generally -60°C), which are difficult to carry out industrially. An attempt to effect the oxidation of compound of formula (**V**) to aldehyde (**II**) was also made using DMSO activated with different activating agents and under temperature conditions more easily reproducible in the industry. The more commonly used DMSO activating agents, such as acetic or trifluoroacetic anhydride and cyanuryl chloride, provided, however, poor results. This route in fact involves formation of too reactive intermediates, even at -10°C, and undesired by-products always form during the oxidation reaction.

There is therefore the need for an alternative process for the preparation of a compound of formula (**II**), which overcomes the drawbacks reported above and can be carried out on an industrial scale.

It has now been found that oxidation of the hydroxyl function of a compound of formula (**V**), when carried out with DMSO activated with an activating agent selected for example from P₂O₅, the pyridine/SO₃ complex and dicyclohexylcarbodiimide (DCC), preferably P₂O₅, can be surprisingly and advantageously carried out at a reaction temperature comprised for example between 0°C and 25°C. The reaction is in fact extremely efficient and cost saving, and the recovery of the product from the reaction mixture is very simple, as no organic by-products are formed, particularly when using P₂O₅.

It is therefore a further object of the invention a process for the preparation of a compound of formula (**II**) comprising the oxidation of a compound of formula (**V**) with DMSO activated with an activating agent selected for example from P₂O₅, the pyridine/SO₃ complex and dicyclohexylcarbodiimide (DCC), preferably P₂O₅.

The oxidation reaction can be carried out treating a compound of formula (**V**) with an at least stoichiometric amount of DMSO and treating the obtained mixture with the DMSO activating agent. The molar ratio of DMSO activating agent to a compound of formula (**V**) is typically comprised between about 1:1 and 8:1, preferably between about 1:1 and 3:1. The reaction mixture is kept under stirring for a time ranging from about 10 minutes to an hour, then treated with a base and left to react for the time necessary to complete the oxidation, usually less than 10 hours. A base can be organic or inorganic. A base is preferably a tertiary amine, in particular triethylamine, diisopropylethylamine, diazabicycloundecene or diazabicyclooctane.

The oxidation reaction can be carried out at a temperature range comprised between about -10°C and 50°C, preferably between about 0°C and 25°C.

The oxidation reaction is preferably carried out in the presence of a solvent, selected from for example a dipolar aprotic solvent, typically dimethylformamide, dimethylacetamide, acetonitrile, dimethylsulfoxide; an ether, typically tetrahydrofuran, dioxane or methyl-tert.butyl ether; a chlorinated solvent, typically dichloromethane; an apolar solvent, typically toluene or hexane; an ester, typically ethyl acetate, isopropyl acetate or butyl acetate; or a mixture of two or more, preferably two or three, of said solvents. The solvent is preferably dichloromethane.

The compound of formula (**V**) can be obtained by reduction of the triple bond of the compound (**VI**) 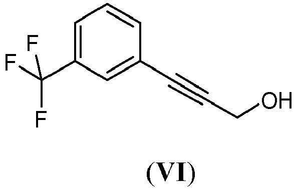 for example by catalytic hydrogenation, by known methods.

The compound (**VI**) can be prepared from 3-bromo-benzotrifluoride (**VII**) 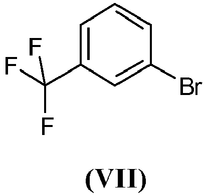 according to known methods, for example according to what reported in EP 2022777.

The following examples illustrate the invention.

### Example 1: Synthesis of 3-[3-(trifluo romethyl)phenyl]propanol (V)

50 g of 3-bromo benzotrifluoride (0.22 mol, 31 ml) is dissolved in 75 ml of triethylamine and 25 ml of dimethylacetamide under nitrogen atmosphere. The mixture is heated to 50°C, then 340 mg (1.76 mmol) of copper (I) iodide, 155 mg (0.88 mmol) of palladium (II) chloride and 930 mg (3.55 mmol) of triphenylphosphine are added. The mixture is adjusted to 70°C, then 16 ml (16 g, 0.29 mol) of propargyl alcohol is slowly dropped therein. After 17 hours, the reaction mixture is diluted with toluene and filtered. The filtrate is washed in succession with a 1N HCl aqueous solution, a NaHCO₃ aqueous saturated solution and water. The toluene phase is then concentrated under reduced pressure and the resulting compound (**VI**) is dissolved in methanol (200 ml) and treated with 13.0 g of 5% Pd/C (containing about 49.0% water). The mixture is treated with hydrogen under atmosphere pressure and room temperature for 3 days and subsequently filtered through Celite. The resulting solution is concentrated under reduced pressure to afford 39.5 g of crude compound (**V**). The resulting compound (**V**) can be directly used in the subsequent reaction or purified by distillation and recovered as a colourless oil (b.p.: 58-60°C, 1.5 mbars or 82-86°C, 6-7 mbars).

¹H NMR (300 MHz, CDCl₃), ppm: 7.46-7.38 (m, 4H), 3.68 (t, 2H, J 6.3 Hz), 2.78 (t, 2H, J 7.7 Hz), 1.90 (m, 2H).

### Example 2: Synthesis of 3-[3-(trifluoromethyl)phenyl]-propionaldehyde (II)

A solution of compound (**V**) (1.0 g, 4.90 mmol) in dichloromethane (20 ml) is cooled in water / ice bath, treated in succession with DMSO (770 mg, 9.80 mmol) and P₂O₅ (1.39 g, 9.80 mmol) and left under stirring for 30 minutes, while temperature raises to 20°C. The reaction mixture is then cooled in water / ice bath and triethylamine (2.4 ml, 17.15 mmol). The resulting solution is kept under stirring while temperature raises to 20°C. After one hour the mixture is treated with 5% HCl, the phases are separated and the organic one is further washed with 5% HCl. The organic phase is then washed with brine, dried over Na₂SO₄, filtered and concentrated under reduced pressure, to afford 0.99 g of the aldehyde of formula (**II**) in quantitative yield.

¹H NMR (300 MHz, CDCl₃), ppm: 9.83 (t, 1H, J 0.9 Hz), 7.48-7.38 (m, 4H), 3.02 (t, 2H, J 7.2 Hz), 2.82 (m, 2H).

### Example 3: Synthesis of compound (I): Cinacalcet hydrochloride

A solution of compound (**II**) (1.0 g, 4.95 mmol) and (*R*)-1-(1-naphthyl)ethylamine hydrochloride (**III**) (1.03 g, 4.95 mmol) in methanol (10 ml), kept under stirring at 20°C, is treated with solid NaBH(OAc)₃ (2.10 g, 9.89 mmol) in portions. After 2 hours the suspended solid is filtered and the solution is concentrated under reduced pressure. The residue is taken up into toluene and washed first with 1% HCl, then with 5% NaOH. The phases are separated and the organic one is concentrated under reduced pressure to afford a residue substantially consisting of Cinacalcet base.

¹H NMR (300 MHz, CDCl₃), ppm: 8.20 (d, 1H, J 7.5 Hz), 7.88 (m, 1H), 7.76 (d, 1H, J 8.1 Hz), 7.66 (d, 1H, J 7.2 Hz), 7.55-7.43 (m, 5H), 7.38-7.30 (m, 2H), 4.63 (q, 1H, J 6.6 Hz), 2.80-2.56 (m, 4H), 1,84 (m, 2H), 1.50 (d, 2H, J 6.6 Hz).

The residue is then diluted in ethyl acetate and the resulting solution is cooled to 10°C, then hydrochloric acid gas is bubbled therein until marked acidity (pH 2). After one hour, the resulting solid is filtered (1.56 g) to obtain 80% yield and 99% purity. The solid is then redissolved in isopropanol under reflux and recrystallized by slowly cooling the resulting solution. Cinacalcet hydrochloride crystals are washed with water and dried, to afford 1.40 g of product, in 90% yield e purity higher than 99.5%. The resulting product has an XRPD wherein the more intense peaks characterizing correspond to those reported in figure 1 of WO 06/127833, corresponding to the form I as defined therein. The size of the crystals is characterized by a D₅₀ value between about 25 and 250 µm.

¹H NMR (300 MHz, DMSO-d₆), ppm: 9.80 (bs, 1H), 9.30 (bs, 1H), 8.22 (d, 1 H, J 7.8 Hz), 8.02-7.94 (m, 3H), 7.62-7.44 (m, 7H), 5.28 (q, 1H, J 6.6 Hz), 2.96-2.92 (m, 1H), 2.78-2.66 (m, 3H), 2.04-1.95 (m, 2H), 1.67 (d, 2H, J 6.6 Hz).

## Claims

1. A process for preparing a compound of formula (**I**) or a salt thereof 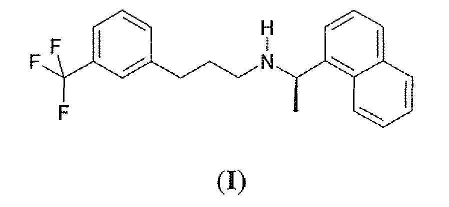 comprising the reductive amination of a compound of formula (**II**) 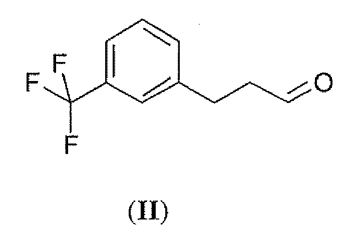 by reaction with (R)-1-(1-naphthyl)ethylamine of formula (**III**) or a salt thereof 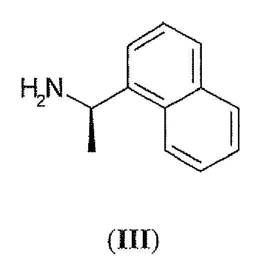 in the presence of a selective reducing agent of the imines **characterized in that** the compound of formula (**II**) is prepared by oxidation of a compound of formula (V) 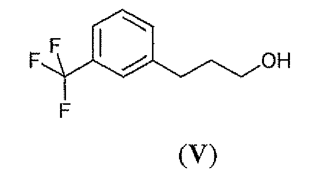 with dimethyl sulphoxide (DMSO) activated with P₂O₅.

2. Process according to claim 1, wherein the oxidation reaction is carried out at a temperature comprised between about -10°C and 50°C.

3. Process according to claim 2, wherein the molar ratio between the activating agent of DMSO and a compound of formula (**V**) is comprised between about 1:1 and 8:1.

4. Process according to claims from 1 to 3, wherein the oxidation reaction is carried out in presence of a solvent, selected from a dipolar aprotic solvent; an ether; a chlorinated solvent; an apolar solvent; an ester and a mixture of two or more of said solvents.

5. Process according to claim 4, wherein the solvent is dichloromethane.

6. Process according to claim 1, wherein the selective reducing agent is selected from sodium triacetoxyborohydride; the InCl₃/triethylsilane system; the Ti(iPrO)₄/polymethylhydrosiloxane system; Bu₂SnC1H; Bu₂SnIH; the PhMe₂SiH/B(C₆F₅)₃ system; Zn(BH₄)₂/silica gel; NiCl₂/NaBH₄; and hydrogen in catalytic hydrogenation conditions.

7. Process according to claim 6, wherein the selective reducing agent is sodium triacetoxyborohydride.

8. Process according to claim 1, wherein the reductive amination is carried out in presence of a solvent selected from an aprotic dipolar solvent; an ether; a straight or branched C₁-C₆ alkanol; and a C₁-C₆ carboxylic acid or a mixture of two or more of said solvents; or in water or in an aqueous solution of a protic organic or mineral acid or a mixture thereof with one or more of said solvents.

9. Process according to claims 1-8, further comprising the preparation of a salt of a compound of formula (**I**), by reacting a compound of formula (**I**), as free base, with a protic organic or mineral acid in presence of ethyl acetate.

10. Process according to claim 8, further comprising the recrystallization of a salt of a compound of formula (**I**), from a solvent in which said salt is poorly soluble at a temperature comprised between about -10°C and 20°C, whereas the same salt is soluble at a temperature comprised between about 20°C and the reflux temperature of the crystallization solvent.

11. Process according to claims 9 or 10, wherein the salt of a compound of formula (**I**) is the hydrochloride salt.

12. Process according to claim 10, wherein the crystallization solvent is selected from isopropanol, ethyl acetate and a mixture acetonitrile/water.

## Patentansprüche

1. Verfahren zur Herstellung einer Verbindung der Formel (I) oder eines Salzes davon 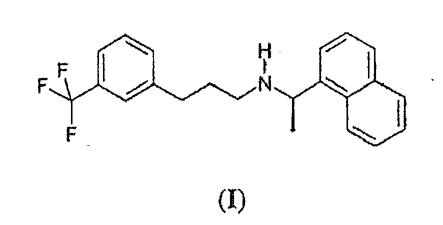 umfassend die reduktive Aminierung einer Verbindung der Formel (II) 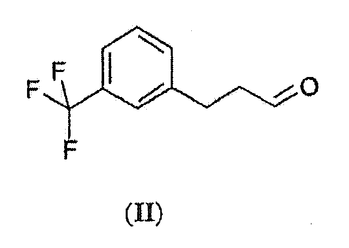 durch Reaktion mit (R)-1-(1-Naphthyl)ethylamin der Formel (III) oder einem Salz davon 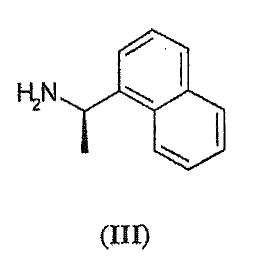 in Gegenwart eines selektiven Reduktionsmittels für Imine, dadurch charakterisiert, dass die Verbindung der Formel (II) durch Oxidation einer Verbindung der Formel (V) 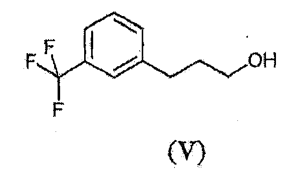 mit Dimethylsulfoxid (DMSO) hergestellt wird, das mit P₂O₅ aktiviert ist.

2. Verfahren nach Anspruch 1, wobei die Oxidationsreaktion bei einer Temperatur zwischen etwa -10°C und 50°C durchgeführt wird.

3. Verfahren nach Anspruch 2, wobei das molare Verhältnis zwischen dem Aktivierungsmittel von DMSO und einer Verbindung der Formel (V) zwischen etwa 1 : 1 und 8 : 1 liegt.

4. Verfahren nach den Ansprüchen 1 bis 3, wobei die Oxidationsreaktion in Gegenwart eines Lösungsmittels durchgeführt wird, das ausgewählt wird aus einem dipolaren aprotischen Lösungsmittel; einem Ether; einem chlorierten Lösungsmittel; einem apolaren Lösungsmittel; einem Ether und einem Gemisch von zwei oder mehr dieser Lösungsmittel.

5. Verfahren nach Anspruch 4, wobei das Lösungsmittel Dichlormethan ist.

6. Verfahren nach Anspruch 1, wobei das selektive Reduktionsmittel ausgewählt wird aus Natriumtriacetoxyborhydrid; dem InCl₃/Triethylsilan-System; dem Ti(iPrO)₄/Polymethylhydrosiloxan-System; Bu₂SnClH; Bu₂SnlH; dem PhMe₂SiH/B(C₆F₅)₃-System; Zn(BH₄)₂/Silikagel; NiCl₂/NABH₄; und Wasserstoff unter katalytischen Hydrierungsbedingungen.

7. Verfahren nach Anspruch 6, wobei das selektive Reduktionsmittel Natriumtriacetoxyborhydrid ist.

8. Verfahren nach Anspruch 1, wobei die reduktive Aminierung in Gegenwart eines Lösungsmittels durchgeführt wird, was ausgewählt wird aus einem aprotischen bipolaren Lösungsmittel; einem Ether; einem geradkettigen oder verzweigtem (C₁-C₆)Alkanol; und einer (C₁-C₆)Carbonsäure oder einem Gemisch von zwei oder mehr dieser Lösungsmittel; oder in Wasser oder in einer wässrigen Lösung einer protischen organischen oder mineralischen Säure oder einem Gemisch davon mit einem oder mehr dieser Lösungsmittel.

9. Verfahren nach den Ansprüchen 1 bis 8, darüber hinaus umfassend die Herstellung eines Salzes der Verbindung der Formel (I) durch Umsetzung einer Verbindung der Formel (I), als freie Base, mit einer protischen organischen oder mineralischen Säure in Gegenwart von Ethylacetat.

10. Verfahren nach Anspruch 8, darüber hinaus umfassend die Umkristallisation eines Salzes der Verbindung der Formel (I) aus einem Lösungsmittel, in dem dieses Salz bei einer Temperatur zwischen etwa -10°C und 20°C schlecht löslich ist, wobei dasselbe Salz bei einer Temperatur zwischen etwa 20°C und der Rückflusstemperatur des Kristallisationslösungsmittels löslich ist.

11. Verfahren nach den Ansprüchen 9 oder 10, wobei das Salz einer Verbindung der Formel (I) das Hydrochloridsalz ist.

12. Verfahren nach Anspruch 10, wobei das Kristallisationslösungsmittel ausgewählt wird aus Isopropanol, Ethylacetat und einem Gemisch Acetonitril/Wasser.

## Revendications

1. Procédé de préparation d'un composé de formule (I) ou d'un sel correspondant 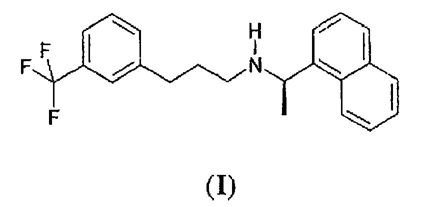 comprenant l'amination réductrice d'un composé de formule (II) 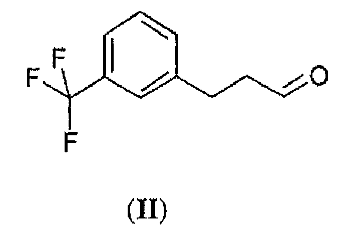 par réaction avec de la (R)-1-(1-naphtyl)éthylamine de formule (III) ou un sel correspondant 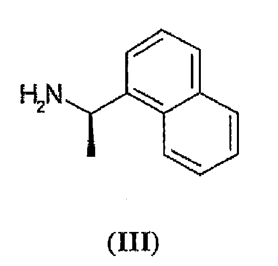 en présence d'un agent de réduction sélectif des imines, **caractérisé en ce que** le composé de formule (II) est préparé par oxydation d'un composé de formule (V) 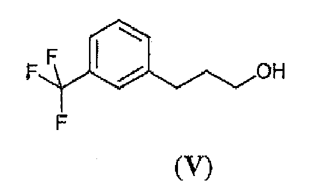 avec du diméthylsulfoxyde (DMSO) activé par du P₂O₅.

2. Procédé selon la revendication 1, la réaction d'oxydation étant effectuée à une température comprise entre environ -10°C et 50°C.

3. Procédé selon la revendication 2, le rapport molaire entre l'agent d'activation de DMSO et un composé de formule (V) étant compris entre environ 1:1 et 8:1.

4. Procédé selon les revendications 1 à 3, la réaction d'oxydation étant effectuée en présence d'un solvant choisi parmi un solvant aprotique dipolaire ; un éther ; un solvant chloré ; un solvant apolaire ; un ester et un mélange de deux desdits solvants ou plus.

5. Procédé selon la revendication 4, le solvant étant le dichlorométhane.

6. Procédé selon la revendication 1, l'agent de réduction sélectif étant choisi parmi le triacétoxyborohydrure de sodium ; le système InCl₃/triéthylsilane ; le système Ti(iPrO)₄/polyméthylhydrosiloxane ; le Bu₂SnClH ; le Bu₂SnIH ; le système PhMe₂SiH/B(C₆F₅)₃ ; Zn(BH₄)₂/gel silicique ; NiCl₂/NaBH₄ ; et l'hydrogène dans des conditions d'hydrogénation catalytique.

7. Procédé selon la revendication 6, l'agent de réduction sélectif étant le triacétoxyborohydrure de sodium.

8. Procédé selon la revendication 1, l'amination réductrice étant effectuée en présence d'un solvant choisi parmi un solvant dipolaire aprotique ; un éther ; un C₁-C₆-alcanol linéaire ou ramifié ; et un acide C₁-C₆-carboxylique ou un mélange de deux desdits solvants ou plus ; ou dans de l'eau ou dans une solution aqueuse d'un acide organique ou minéral protique ou un mélange correspondant avec un ou plusieurs desdits solvants.

9. Procédé selon les revendications 1-8, comprenant en outre la préparation d'un sel d'un composé de formule (I) par la réaction d'un composé de formule (I), sous forme de base libre, avec un acide organique ou minéral protique en présence d'acétate d'éthyle.

10. Procédé selon la revendication 8, comprenant en outre la recristallisation d'un sel d'un composé de formule (I) à partir d'un solvant dans lequel ledit sel est peu soluble à une température comprise entre environ -10°C et 20°C, alors que le même sel est soluble à une température comprise entre environ 20°C et la température de reflux du solvant de cristallisation.

11. Procédé selon l'une quelconque des revendications 9 ou 10, le sel d'un composé de formule (I) étant le sel chlorhydrate.

12. Procédé selon la revendication 10, le solvant de cristallisation étant choisi parmi l'isopropanol, l'acétate d'éthyle et un mélange acétonitrile/eau.
